# EUROPÄISCHE PATENTSCHRIFT

(11) **EP 0 636 115 B1**
(45) Veröffentlichungstag und Bekanntmachung des Hinweises auf die Patenterteilung: **14.08.1996**
(21) Anmeldenummer: 93922323.6
(22) Anmeldetag: 13.04.1993
(51) Int. Cl.: C07C 15/52, A61K 31/015

(54) **NEUE BIS-PHENYL-HEXENE**
NOVEL BIS-PHENYL-HEXENES
NOUVEAUX BIS-PHENYL-HEXENES

(30) Priorität: 15.04.1992 DE 4212628
(43) Veröffentlichungstag der Anmeldung: 01.02.1995
(73) Patentinhaber: METZLER, Manfred, D-67663 Kaiserslautern (DE); PECHAN, Reinhard, D-81925 München (DE)
(72) Erfinder: METZLER, Manfred, D-67663 Kaiserslautern (DE); PECHAN, Reinhard, D-81925 München (DE)
(74) Vertreter: Huber, Bernhard, Dipl.-Chem.
(86) Internationale Anmeldenummer: EP9300892
(87) Internationale Veröffentlichungsnummer: WO9322262

(56) Entgegenhaltungen:
- CHEMICAL ABSTRACTS, vol. 77, no. 3, 17. Juli 1972, Columbus, Ohio, US; abstract no. 14579x, N. OWEN et al: "Papillomatous growths on internal genitalia of bitches administred the synthetic estrogen trans-4,4'-dimethyl-alpha,alpha'-diethylstilbene", Seite 85-86

## Beschreibung

Die vorliegende Erfindung betrifft neue chemische Verbindungen aus der Gruppe der Bis-phenyl-hex-3-ene, ein Verfahren zu ihrer Herstellung und ihre Anwendung als Therapeutika.

Einer Vielzahl von Erkrankungen des Menschen liegt ein unkontrolliertes Wachstum von Körperzellen zugrunde. Diese Proliferationsstörung kann zu einer benignen Entartung, z.B. Psoriasis vulgeris bei Keratinozyten oder β-Thalassämie bei Erythrozyten oder aber auch zu einer malignen Entartung, z.B. bei allen Formen von Krebs, führen.

Das Ziel einer Krebstherapie ist die vollständige Zerstörung, zumindest aber eine signifikante Wachstumshemmung der Tumorzellen. Neben chirurgischen Maßnahmen und Strahlentherapie stehen heute auch Chemotherapeutika, z.B. 5-Fluoruracil, Cytosin-Arabinosid etc., zur Behandlung von Krebspatienten zur Verfügung. Ein Nachteil bei der Verwendung dieser Chemotherapeutika besteht jedoch darin, daß sie neben den Tumorzellen auch gesunde Bereiche des Organismus schädigen können. Daher wurde seit mehreren Jahren nach weniger aggressiven Behandlungsmethoden gesucht. So erbrachte die Anwendung von Tamoxifen bei Mammakarzinomen erste vielversprechende Resultate (Jordan, Antiestrogens in Cancer Treatment, in: Stoll, B.A., Endocrine Management of Cancer, Karger, Basel (1988), 57-65). Bis heute ist jedoch eine Vielzahl von Krebserkrankungen (z.B. Kolon- und Pankreaskarzinom) noch nicht in ausreichendem Umfang durch Medikamente therapierbar (Cohen et al., Colorectal Cancer, und Brennan et al., Cancer of the Pancreas in: DeVita, V.T., Helman, S. und Rosenberg, St. A., Cancer, Lippincott Co., 3rd Edition (1989)).

Aus Norris et al. (Toxikolgy and Applied Pharmacology 21 (1972), 582-585) ist bekannt, daß das synthetische Östrogen trans-4,4'-Dimethyl-α,α'-diethylstilben (DMES) (3,4-Bis(4-methylphenyl)-3-hexen) bei Hunden ein Papillomawachstum fördert bzw. verursacht. Im Unterschied zur berichteten Wirkung von Diethylstilbestrol, das verglichen mit DMES Hydroxy- anstelle von Methylgruppen aufweist, waren die Wucherungen gutartig und bildeten sich nach dem Absetzen der Verabreichung zurück. Weitere Diethylstilbenderivate oder eine wachstumsinhibierende Wirkung derartiger Substanzen auf entartete Zellen ist Norris et al. nicht zu entnehmen.

Als eine der Hauptursachen der Krebsentstehung gelten Fehler bei der Expression von sogenannten Onkogenen, wie etwa myc oder ras (Tabin et al., Nature, 300 (1982), 143-149). Ein wesentlicher Regulationsmechanismus der Genexpression - auch bei Onkogenen - ist der Methylierungsgrad der DNA (Doerfler, J. gen. Virol. 57 (1981), 1-20). So konnte am Beispiel der ras- und myc-Onkogene eine Hypomethylierung im exprimierten kanzerogenen Status nachgewiesen werden (Feinberg et al., Biophys. Res. Comm. 111 (1983), 47-54; Cheah et al., J. Nat. Cancer Inst. 73 (1984), 1057-1061). Weiterhin ist bekannt, daß durch eine gezielte Steuerung der DNA-Methylierung sowohl das Wachstum als auch die korrekte somatische Funktion von entarteten Zellen reguliert werden kann. Dies wurde von Ley et al. (DNA-methylation and globin gene expression in patients treated with 4-azacytidine, in: Globin Gene Expression and Hematopoietic Differentiation, Alan P. Liss, N.Y. (1983), 457-474) gezeigt. Ein weiterer direkter Zusammenhang zwischen der Methylierung von DNA und Störungen im zentralen Nervensystem wurde bei HIV-positiven Patienten gefunden (Keating et al., Lancet 337 (1991), 935-939).

Ferner konnte bei in vitro Zellkulturversuchen ein direkter Zusammenhang zwischen maligner Transformation und DNA-Hypomethylierung einerseits sowie zwischen konzentrationsabhängiger Wachstumsinhibierung von Tumorzellen (aus Mensch und Tier) und DNA-Hypermethylierung andererseits nachgewiesen werden (Pechan (1987), Dissertation, Universität Würzburg).

Eine Aufgabe der vorliegenden Erfindung war die Bereitstellung neuer Verbindungen, die eine pharmazeutische Wirksamkeit bei der Behandlung von Erkrankungen zeigen, die im Zusammenhang mit entartetem Zellwachstum, d.h. Störungen der Zellproliferation, stehen.

Die erfindungsgemäße Aufgabe wird durch ein 3,4-Bis(4-X-phenyl)-3-hexen gelöst, bei dem die Gruppen X jeweils gegebenfalls substituierte Alkylreste mit 2 bis 4 Kohlenstoffatomen, Cycloalkylreste mit bis zu 4 Kohlenstoffatomen, Alkenyl- oder Alkinylreste mit bis zu 10 Kohlenstoffatomen oder gegebenenfalls substituierte Aralkylreste mit 1 bis 10 nicht aromatischen Kohlenstoffatomen darstellen.

Vorzugsweise sind die Gruppen X gegebenenfalls substituierte Alkenyl- oder Alkinylreste mit jeweils bis zu 5 Kohlenstoffatomen.

Besonders bevorzugt sind die Gruppen X in der erfindungsgemäßen Verbindung Alkylreste mit 2 bis 4 Kohlenstoffatomen, am meisten bevorzugt sind die Gruppen X Ethyl-, n-Propyl-, iso-Propyl-, sek.-Butyl- oder tert.-Butylreste.

Der Begriff "Alkylrest" gemäß vorliegender Erfindung umfaßt aliphatische oder cycloaliphatische Kohlenwasserstoffreste, die gegebenenfalls aber noch einen oder mehrere inerte Substituenten (z.B. Halogengruppen oder Alkoxygruppen) aufweisen können.

Ein "Alkenylrest" gemäß vorliegender Erfindung ist ein gegebenenfalls (inert) substituierter Kohlenwasserstoffrest mit einer oder mehreren C=C Bindungen, z.B. ein Vinyl- oder ein Allylrest. Eine analoge Definition gilt für den Begriff "Alkinylrest". Der Begriff "Aralkylrest" bedeutet einen Alkyl-, Alkenyl- oder Alkinylrest, der zusätzlich eine gegebenenfalls substituierte Arylgruppe trägt, z.B. ein Benzylrest.

Die erfindungsgemäßen Verbindungen sind durch ein Verfahren erhältlich, welches dadurch gekennzeichnet ist, daß man ein 1-(4-X-phenyl)-1-propanon (wobei X gemäß obiger Definition ist) in Gegenwart eines Übergangsmetallchlorids, Zinkstaub und einer Base in einem inerten Lösungsmittel umsetzt und die gewünschte Verbindung aus dem Reaktionsgemisch durch Aufreinigung gewinnt.

Das Übergangsmetallchlorid ist vorzugsweise Titantetrachlorid, und die Base ist vorzugsweise Pyridin. Das inerte Lösungsmittel ist vorzugsweise 1,4-Dioxan und die Umsetzung erfolgt günstigerweise bei der Rückflußtemperatur des Lösungsmittels.

Weitere Einzelheiten dieses Verfahrens sind in einer Arbeit von McMurry (J. Org. Chem. 54 (1989), 3748-3749) beschrieben.

Überraschenderweise zeigen die erfindungsgemäßen Substanzen eine signifikante Wachstumsinhibition von Tumorzellen in vitro. Daher ist zu erwarten, daß sich die Verbindungen als Therapeutika zur Behandlung von Erkrankungen eignen, die im Zusammenhang mit entartetem Zellwachstum stehen.

Ein weiterer Gegenstand der vorliegenden Erfindung ist daher eine pharmazeutische Zusammensetzung, die eine oder mehrere erfindungsgemäße Verbindungen als Wirkstoff und gegebenenfalls pharmazeutisch übliche Träger-, Füll-, Verdünnungs-oder/und Hilfsstoffe enthält.

Die erfindungsgemäßen Verbindungen eignen sich für alle Indi kationsgebiete der Human- und Veterinärmedizin, bei denen eine Wachstumshemmung von entarteten Körperzellen oder/und deren Beeinflussung aufgrund einer DNA-Hypermethylierung erfolgen kann. Insbesondere sind hier Krebs, Psoriasis, oder AIDS zu nennen.

Die erfindungsgemäße pharmazeutische Zusammensetzung kann dabei in beliebigen Formulierungen verabreicht werden, welch die erfindungsgemäßen Verbindungen enthalten. Beispiele für geeignete Formulierungen sind etwa Tabletten, Kapseln, Granu late, Lösungen, Salben oder Aerosole. Die Herstellung derartiger Formulierungen ist einem Fachmann auf dem Gebiet der Pharmazie hinreichend bekannt, so daß eine ausführliche Beschreibung nicht erforderlich scheint.

Die folgenden Beispiele dienen zur weiteren Verdeutlichung der Erfindung.

### Beispiel 1

### Synthese von 3,4-Bis(4-alkylphenyl)-3-hexenen nach McMurry (1989, supra)

Zu 200 ml 1,4-Dioxan werden unter Rühren (Eiskühlung, Argonatmosphäre) 108 mmol Titantetrachlorid zugetropft. Dieser Lösung werden in kleinen Portionen 200 mmol Zinkstaub und 8 ml Pyridin zugegeben. Nach langsamer (30 min) Zugabe von 1-(4-Alkylphenyl)-l-propanon wird die Reaktionslösung unter Rückfluß erhitzt (20 h). Nach dem Erkalten wird diese Lösung mit 200 ml 10%iger Kaliumcarbonatlösung versetzt. Die so entstandene Suspension wird mit 50 ml Diethylether ausgeschüttelt, mit Wasser gewaschen und über Magnesiumsulfat getrocknet.

Zur Reinigung wird das Rohprodukt über Kieselgel mit Petrolether/Diethylether (4/1) filtriert und anschließend durch Säulenchromatographie (Kieselgel, Petrolether/Diethylether 2/1) getrennt. Die Reinsubstanz (farblos) kristallisiert in der Kälte nach mehreren Tagen.

Es wurden folgende Substanzen hergestellt:

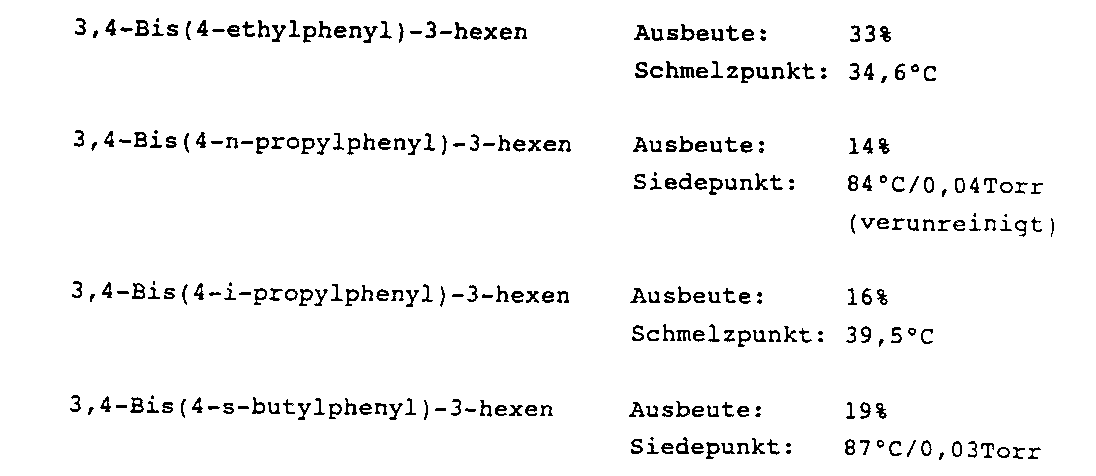

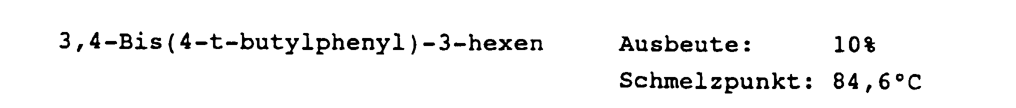

### Beispiel 2

### Wachstumsinhibition von Tumorzellen in vitro

In Zellkulturflaschen (T25, Greiner) wurden 10⁴ bis 10⁵ Tumorzellen in jeweils 3 ml IBR-Medium (15% Fetales Kälberserum, 25 U/ml Penicillin-Streptomycin) ausgesät. Nach einer Anwachsphase von 4h (37°C, 12% CO₂) wurden je 50 µM Testsubstanz in 0.1% DMSO gelöst, sowie 0,1% DMSO (Kontrolle), zugegeben. Als Testsubstanzen wurden 3,4-Bis(4-ethylphenyl)-3-hexen, 3,4-Bis(4-i-propylphenyl)-3-hexen und 3,4-Bis(4-t-butylphenyl)-3-hexen verwendet.

Die Zählung der Zellen (Neubauer-Zählkammer) erfolgte in Abständen von 24 h, über einen Zeitraum von 5 Tagen. Nach 3 Tagen wurde das Medium gewechselt und die Testsubstanzen (siehe Beispiel 1) erneut in gleicher Konzentration zugegeben.

Das Wachstum von HT29 Leberkarzinomzellen (Mensch) konnte um etwa 40 % inhibiert werden, das von in vitro transformierten Hamsterzellen um etwa 30%.

## Patentansprüche

1. Ein 3,4-Bis(4-X-phenyl)-3-hexen, bei dem die Gruppen X jeweils gegebenenfalls substituierte Alkylreste mit 2 bis 4 Kohlenstoffatomen, Cycloalkylreste mit bis zu 4 Kohlenstoffatomen, Alkenyl- oder Alkinylreste mit bis zu 10 Kohlenstoffatomen oder gegebenenfalls substituierte Aralkylreste mit 1 bis 10 nicht aromatischen Kohlenstoffatomen darstellen.

2. Verbindung nach Anspruch 1,
**dadurch gekennzeichnet,**
daß die Gruppen X gegebenenfalls substituierte Alkenyl-oder Alkinylreste mit jeweils bis zu 5 Kohlenstoffatomen darstellen.

3. Verbindungen nach Anspruch 1 oder 2,
**dadurch gekennzeichnet,**
daß die Gruppen X Ethyl-, n-Propyl-, iso-Propyl-, sek.- Butyl- oder tert.-Butylreste sind.

4. Verfahren zur Herstellung einer Verbindung nach einem der Ansprüche 1 bis 3,
**dadurch gekennzeichnet,**
daß man ein 1-(4-X-phenyl)-1-propanon, wobei X wie in Anspruch 1 definiert ist, in Gegenwart eines Übergangsmetalls, Chlorids, Zinkstaub und einer Base in einem inerten Lösungsmittel umsetzt und die gewünschte Verbindung aus dem Reaktionsgemisch durch Aufreinigung gewinnt.

5. Pharmazeutische Zusammensetzung,
**dadurch gekennzeichnet,**
daß sie eine oder mehrere Verbindungen nach einem der Ansprüche 1 bis 3 als Wirkstoff und ggf. pharmazeutisch übliche Träger-, Füll-, Verdünnungs- oder/und Hilfsstoffe enthält.

6. Verwendung einer Verbindung nach einem der Ansprüche 1 bis 3 zur Herstellung einer pharmazeutischen Zusammensetzung zur Behandlung von Erkrankungen, die im Zusammenhang mit entartetem Zellwachstum stehen.

7. Verwendung nach Anspruch 8 zur Behandlung von Krebs, Psoriasis oder AIDS.

## Claims

1. A 3,4-bis(4-X-phenyl)-3-hexene in which the groups X in each case represent optionally substituted alkyl residues with 2 to 4 carbon atoms, cycloalkyl residues with up to 4 carbon atoms, alkenyl or alkinyl residues with up to 10 carbon atoms or optionally substituted aralkyl residues with 1 to 10 non-aromatic carbon atoms.

2. Compound as claimed in claim 1,
**wherein**
the groups X represent, alkenyl or alkinyl residues each with up to 5 carbon atoms which are substituted if desired.

3. Compounds as claimed in claim 1 or 2,
**wherein**
the groups X are ethyl, n-propyl, iso-propyl, sec.-butyl or tert.-butyl residues.

4. Process for the production of a compound as claimed in one of the claims 1 to 3,
**wherein**
a 1-(4-X-phenyl)-1-propanone in which X is defined as in claim 1, is reacted in the presence of a transition metal chloride, zinc dust and a base in an inert solvent and the desired compound is isolated from the reaction mixture by purification.

5. Pharmaceutical composition,
**wherein**
it contains one or several compounds as claimed in one of the claims 1 to 3 as the active substance and if desired, conventional pharmaceutical carrier substances, fillers, diluents or/and auxiliary substances.

6. Use of a compound as claimed in one of the claims 1 to 3 for the production of a pharmaceutical composition for the treatment of diseases which are associated with degenerate cell growth.

7. Use as claimed in claim 8 for the treatment of cancer, psoriasis or AIDS.

## Revendications

1. 3,4-bis(4-X-phényl)-3-hexène dans lequel les groupes X représentent respectivement des radicaux alkyle éventuellement substitués par 2 à 4 atomes de carbone, des radicaux cycloalkyle avec jusqu'à 4 atomes de carbone, des radicaux alcényle ou alkynyle avec jusqu'à 10 atomes de carbone ou éventuellement des radicaux aralkyle éventuellement substitués par 1 jusqu'à 10 atomes de carbone non aromatiques.

2. Composition selon la revendication 1,
caractérisée en ce que les groupes X représentent éventuellement des radicaux alcényle ou alkynyle éventuellement substitués par respectivement jusqu'à 5 atomes de carbone.

3. Composition selon la revendication 1 ou 2,
caractérisée en ce que les groupes X sont des radicaux éthyle, n-propyle, isopropyle, sec-butyle ou tert-butyle.

4. Procédé pour la préparation d'un composé selon l'une des revendications 1 à 3,
caractérisé en ce que l'on met en réaction un 1-(4-X-phényl)-1-propanone, X étant défini comme dans la revendication 1, en présence d'un métal de transition, de chlorure, de poussière de zinc et d'une base dans un solvant inerte et en ce que l'on obtient le composé souhaité à partir du mélange réactionnel par purification.

5. Composition pharmaceutique
caractérisée en ce qu'elle contient un ou plusieurs composés selon l'une des revendications 1 à 3, en tant que principes actifs et éventuellement des auxiliaires et/ou agents de dilution, de remplissage ou de support pharmaceutiquement usuels.

6. Utilisation d'un composé selon l'une des revendications 1 à 3 pour la préparation d'une composition pharmaceutique pour le traitement de maladies qui sont en liaison avec altération de la croissance cellulaire.

7. Utilisation selon la revendication 8 pour le traitement du cancer, du psoriasis ou du sida.
